# EUROPEAN PATENT SPECIFICATION

(11) **EP 3 498 159 B1**
(45) Date of publication and mention of the grant of the patent: **29.09.2021**
(21) Application number: 16914561.2
(22) Date of filing: 31.08.2016
(51) Int. Cl.: A61B 5/021

(54) **METHOD, APPARATUS AND DEVICE FOR DETERMINING BLOOD PRESSURE CALIBRATION PERIOD**
VERFAHREN, VORRICHTUNG UND EINRICHTUNG ZUR BESTIMMUNG EINER BLUTDRUCKKALIBRIERDAUER
PROCÉDÉ, APPAREIL ET DISPOSITIF PERMETTANT DE DÉTERMINER UNE PÉRIODE D'ÉTALONNAGE DE PRESSION ARTÉRIELLE

(43) Date of publication of application: 19.06.2019
(73) Proprietor: Huawei Technologies Co., Ltd., Longgang District Shenzhen, Guangdong 518129 (CN)
(72) Inventor: ZHANG, Anqi, Shenzhen Guangdong 518129 (CN); CHEN, Wenjuan, Shenzhen Guangdong 518129 (CN); XU, Peida, Shenzhen Guangdong 518129 (CN)
(74) Representative: Epping - Hermann - Fischer
(86) International application number: PCT/CN2016/097590
(87) International publication number: WO 2018/039995

(56) References cited:
- CN-A- 1 672 631
- CN-A- 103 632 055
- CN-A- 104 257 371
- CN-A- 104 814 729
- US-A1- 2006 142 663
- US-A1- 2010 081 944
- US-A1- 2010 081 945
- US-A1- 2011 077 486
- US-A1- 2012 136 261

## Description

### TECHNICAL FIELD

Embodiments of the present invention relate to the medical field, and in particular, to a blood pressure calibration period determining method and apparatus, and a device.

### BACKGROUND

Blood pressure is a force that pushes blood to circulate in a blood vessel, can reflect a functional status of human angiocarpy, and is one of important physiological parameters of a human body. Currently, the blood pressure may be measured by using a blood pressure measurement device.

In the prior art, a blood pressure measurement device may measure blood pressure by using a photo plethysmo graphy (PPG, Photo Plethysmo Graphy) signal. When the blood pressure is measured by using the PPG, the blood pressure needs to be measured in a PPG calibration mode to ensure measurement precision. When the blood pressure is measured in the PPG calibration mode, a blood pressure measurement performed by the blood pressure measurement device needs to be calibrated. That is, a correspondence needs to be established between an actual blood pressure value of a tested person and a PPG signal characteristic (for example, a pulse wave velocity (PWV, Pulse Wave Velocity) or a pulse transit time (PTT, Pulse Transit Time)), and saved as calibration information. In a subsequent measurement, the calibration information is used as a sample to calculate a blood pressure value of the tested person with reference to a current PPG signal characteristic of the tested person. However, information that causes a change in the blood pressure of the tested person does not remain unchanged, and a change in the information results in that the original calibration information cannot truly reflect a relationship between a PPG signal characteristic and blood pressure during an actual blood pressure measurement process. Therefore, when the blood pressure measurement device currently measures the blood pressure in the PPG calibration mode, the tested person needs to calibrate, at an interval of a fixed blood pressure calibration period, a blood pressure measurement performed by the blood pressure measurement device.

However, in the prior art, when the blood pressure measurement device is calibrated at an interval of the fixed blood pressure calibration period, a blood pressure measurement result obtained by using the blood pressure measurement device is inaccurate.

Document US 2010/0081944 A1 relates to techniques for non-invasive blood pressure monitoring. Data corresponding to a patient may be received from a hospital information system. The data may include, for example, drug administration data, medical procedure data, medical equipment data, or a combination thereof. Whether a blood pressure monitoring system needs to be recalibrated may be determined, based at least in part on the received data. If it is determined that the blood pressure monitoring system needs to be recalibrated, the recalibration may be performed and at least one blood pressure measurement of the patient may be computed using the recalibrated blood pressure monitoring system.

Document US 2012/0136261 A1 discloses systems and methods for calibrating the calculation of physiological parameters. Two or more calibration techniques may be used to determine a relationship between physiological measurements and a desired physiological parameter, such as a relationship between differential pulse transit time (DPTT) and blood pressure. Different calibration techniques may be used in a serial fashion, one after the other, or in a parallel fashion, with different weights accorded to each calibration technique. When physiological or other changes occur, the calibration data may be stored for later use and new calibration data may be generated.

Document US 2010/0081945 A1 discloses systems and methods for maintaining the calibration of non-invasive blood pressure monitoring devices. Phase components of pulse signals detected by the blood pressure monitoring device are compared to stored baseline phase component values. If the difference exceeds a threshold, the blood pressure monitoring device is recalibrated.

Document US 2011/0077486 A1 relates to systems and methods for analyzing and normalizing signals, such as PPG signals, for use in patent monitoring. The PPG signal may be detected using a continuous non-invasive blood pressure monitoring system and the normalized signals may be used to determine whether a recalibration of the system should be performed.

### SUMMARY

The present invention is defined by the attached set of claims.

Embodiments of the present invention provide a blood pressure calibration period determining method and apparatus, and a device, so as to resolve a prior-art problem that a blood pressure measurement result obtained by using a blood pressure measurement device is inaccurate when the blood pressure measurement device is calibrated at an interval of a fixed blood pressure calibration period.

According to a first aspect, an embodiment of the present invention provides a blood pressure calibration period determining method. The method includes: obtaining, by a blood pressure measurement device, first physiological status information and second physiological status information of a tested person, wherein the first physiological status information is related information that causes a change in blood pressure of the tested person within a first time period after the blood pressure is calibrated, and the second physiological status information is related information that causes a change in the blood pressure of the tested person within a second time period before the blood pressure is calibrated; and determining, by the blood pressure measurement device, a blood pressure calibration period based on a change degree of the first physiological status information relative to the second physiological status information,wherein the related information comprises exercise information which comprises at least one of the following information: exercise intensity, exercise duration, or an exercise regularity,wherein obtaining, by the blood pressure measurement device, the exercise information of the tested person comprises:determining, by the blood pressure measurement device, the exercise information based on information output by an acceleration sensor and/or a gyro sensor (603) that are/is disposed on the blood pressure measurement device, and the blood pressure measurement device further includes a communications module, so that the blood pressure measurement device communicates bidirectionally with one or more other electronic apparatuses or servers, the communications module supports a near field communications protocol, or near field communication, or a communications protocol used by a network.

According to the blood pressure calibration period determining method provided in this implementation, the blood pressure measurement device determines the blood pressure calibration period based on the change degree of the related information that causes a change in the blood pressure of the tested person within the first time period after the blood pressure is calibrated relative to the related information that causes a change in the blood pressure of the tested person within the second time period before the blood pressure is calibrated. Therefore, the blood pressure measurement device may adjust the blood pressure calibration period based on the related information that causes a change in the blood pressure of the tested person. Compared with a method of using a fixed blood pressure calibration period, this method implements more appropriate selection of a blood pressure calibration period, thereby improving blood pressure measurement accuracy of the blood pressure measurement device.

In a possible design, the determining, by the blood pressure measurement device, a blood pressure calibration period based on a change degree of the first physiological status information relative to the second physiological status information includes: determining, by the blood pressure measurement device, whether the change degree of the first physiological status information relative to the second physiological status information is greater than or equal to a preset threshold; and if the change degree is greater than or equal to the preset threshold, determining that the blood pressure calibration period is a first period, where the first period is less than a preset blood pressure calibration period of the blood pressure measurement device.

In a possible design, the method further includes: if the change degree is less than the preset threshold, determining that the blood pressure calibration period is a second period, where the second period is greater than or equal to the preset blood pressure calibration period.

According to the blood pressure calibration period determining method provided in this implementation, when determining that the change degree of the first physiological status information relative to the second physiological status information is greater than or equal to the preset threshold, the blood pressure measurement device determines that the blood pressure calibration period is the first period that is less than the preset blood pressure calibration period, or when determining that the change degree of the first physiological status information relative to the second physiological status information is less than the preset threshold, the blood pressure measurement device determines that the blood pressure calibration period is the second period that is greater than or equal to the preset blood pressure calibration period. Therefore, the blood pressure measurement device may adjust the blood pressure calibration period based on the related information that causes a change in the blood pressure of the tested person, thereby improving blood pressure measurement accuracy of the blood pressure measurement device.

In a possible design, the related information further includes at least one of the following information: medication information, sleep information, or environment information.

In a possible design, the medication information includes a drug type and drug use information, and the drug use information includes at least one of the following information: a drug dose, an efficacy time, or a medication regularity.

In a possible design, obtaining, by the blood pressure measurement device, the medication information of the tested person includes: obtaining, by the blood pressure measurement device, the medication information from a medication reminding application; or determining, by the blood pressure measurement device, the medication information based on a user input.

According to the blood pressure calibration period determining method provided in this implementation, the blood pressure measurement device determines the blood pressure calibration period based on medication information that causes a change in the blood pressure of the tested person within the first time period after the blood pressure is calibrated and medication information that causes a change in the blood pressure of the tested person within the second time period before the blood pressure is calibrated. Therefore, the blood pressure measurement device may adjust the blood pressure calibration period based on the medication information that causes a change in the blood pressure, thereby improving blood pressure measurement accuracy of the blood pressure measurement device.

According to the invention the exercise information includes at least one of the following information: exercise intensity, exercise duration, or an exercise regularity.

According to the invention, obtaining, by the blood pressure measurement device, the exercise information of the tested person includes: determining, by the blood pressure measurement device, the exercise information based on information output by an acceleration sensor and/or a gyro sensor that are/is disposed on the blood pressure measurement device.

According to the blood pressure calibration period determining method provided in this implementation, the blood pressure measurement device determines the blood pressure calibration period based on exercise information that causes a change in the blood pressure of the tested person within the first time period after the blood pressure is calibrated and exercise information that causes a change in the blood pressure of the tested person within the second time period before the blood pressure is calibrated. Therefore, the blood pressure measurement device may adjust the blood pressure calibration period based on the exercise information that causes a change in the blood pressure, thereby improving blood pressure measurement accuracy of the blood pressure measurement device.

In a possible design, the sleep information includes a sleep time and/or sleep quality.

In a possible design, obtaining, by the blood pressure measurement device, the sleep information of the tested person includes: determining, by the blood pressure measurement device, the sleep information based on information output by an acceleration sensor, a gyro sensor, and a photo plethysmo graphy PPG sensor that are disposed on the blood pressure measurement device.

According to the blood pressure calibration period determining method provided in this implementation, the blood pressure measurement device determines the blood pressure calibration period based on sleep information that causes a change in the blood pressure of the tested person within the first time period after the blood pressure is calibrated and sleep information that causes a change in the blood pressure of the tested person within the second time period before the blood pressure is calibrated. Therefore, the blood pressure measurement device may adjust the blood pressure calibration period based on the sleep information that causes a change in the blood pressure, thereby improving blood pressure measurement accuracy of the blood pressure measurement device.

In a possible design, the environment information includes human body environment information and/or external environment information.

In a possible design, the external environment information includes at least one of the following information: an ambient temperature, an altitude, contact pressure between a measured part and a sensor of the blood pressure measurement device, or ambient light intensity.

According to the blood pressure calibration period determining method provided in this implementation, the blood pressure measurement device determines the blood pressure calibration period based on environment information that causes a change in the blood pressure of the tested person within the first time period after the blood pressure is calibrated and environment information that causes a change in the blood pressure of the tested person within the second time period before the blood pressure is calibrated. Therefore, the blood pressure measurement device may adjust the blood pressure calibration period based on the environment information that causes a change in the blood pressure, thereby improving blood pressure measurement accuracy of the blood pressure measurement device.

In a possible design, a priority of the medication information is higher than priorities of the exercise information, the sleep information, and the environment information.

According to a second aspect, an embodiment of the present invention provides a blood pressure measurement device, including a processor and a memory, wherein wherein the processor executes an instruction stored in the memory to perform the following operations: obtaining first physiological status information and second physiological status information of a tested person, wherein the first physiological status information is related information that causes a change in blood pressure of the tested person within a first time period after the blood pressure is calibrated, and the second physiological status information is related information that causes a change in the blood pressure of the tested person within a second time period before the blood pressure is calibrated; and determining a blood pressure calibration period based on a change degree of the first physiological status information relative to the second physiological status information, wherein the related information comprises exercise information, which comprises at least one of the following information: exercise intensity, exercise duration, or an exercise regularity, wherein obtaining, by the blood pressure measurement device, the exercise information of the tested person comprises: determining, by the blood pressure measurement device, the exercise information based on information output by an acceleration sensor and/or a gyro sensor that are/is disposed on the blood pressure measurement device, and the blood pressure measurement device further includes a communications module, so that the blood pressure measurement device is configured to communicate bidirectionally with one or more other electronic apparatuses or servers, the communications module is configured to support a near field communications protocol, or near field communication, or a communications protocol used by a network.

In a possible design, that the processor determines a blood pressure calibration period based on a change degree of the first physiological status information relative to the second physiological status information specifically includes:
determining whether the change degree of the first physiological status information relative to the second physiological status information is greater than or equal to a preset threshold; and
if the change degree is greater than or equal to the preset threshold, determining that the blood pressure calibration period is a first period, where the first period is less than a preset blood pressure calibration period of the blood pressure measurement device.

In a possible design, the processor is further configured to:
if the change degree is less than the preset threshold, determine that the blood pressure calibration period is a second period, where the second period is greater than or equal to the preset blood pressure calibration period.

In a possible design, the related information further includes at least one of the following information:
medication information, sleep information, or environment information.

In a possible design, the medication information includes a drug type and drug use information, and the drug use information includes at least one of the following information: a drug dose, an efficacy time, or a medication regularity.

In a possible design, the blood pressure measurement device further includes a communications module; and
that the processor obtains the medication information of the tested person specifically includes:
obtaining the medication information from a medication reminding application by using the communications module.

In a possible design, the blood pressure measurement device further includes an input module; and
that the processor obtains the medication information of the tested person specifically includes:
or, determining the medication information based on a user input obtained by using the input module.

According to the present invention, the exercise information includes at least one of the following information: exercise intensity, exercise duration, or an exercise regularity.

According to the invention the blood pressure measurement device further includes an acceleration sensor and a gyro sensor; and
that the processor obtains the exercise information of the tested person specifically includes:
determining the exercise information based on information output by the acceleration sensor and/or the gyro sensor.

In a possible design, the blood pressure measurement device further includes an input module; and
that the processor obtains the exercise information of the tested person specifically includes: determining the exercise information based on a user input obtained by using the input module.

In a possible design, the sleep information includes a sleep time and/or sleep quality.

In a possible design, the blood pressure measurement device further includes an acceleration sensor, a gyro sensor, and a photo plethysmo graphy PPG sensor; and
that the processor obtains the sleep information of the tested person specifically includes:
determining the sleep information based on information output by the acceleration sensor, the gyro sensor, and the PPG sensor.

In a possible design, the environment information includes human body environment information and/or external environment information.

In a possible design, the external environment information includes at least one of the following information: an ambient temperature, an altitude, contact pressure between a measured part and a sensor of the blood pressure measurement device, or ambient light intensity.

In a possible design, a priority of the medication information is higher than priorities of the exercise information, the sleep information, and the environment information.

For beneficial effects of the blood pressure calibration period determining apparatus provided in the third aspect and the possible implementations of the third aspect, refer to beneficial effects brought by the first aspect and the possible implementations of the first aspect, and details are not described herein.

### BRIEF DESCRIPTION OF DRAWINGS

To describe the technical solutions in the embodiments of the present invention or in the prior art more clearly, the following briefly describes the accompanying drawings required for describing the embodiments or the prior art. Apparently, the accompanying drawings in the following description show some embodiments of the present invention, and persons of ordinary skill in the art may still derive other drawings from these accompanying drawings without creative efforts.
FIG. 1 is a flowchart of Embodiment 1 of a blood pressure calibration period determining method according to the present invention;
FIG. 2 is a flowchart of Embodiment 2 of a blood pressure calibration period determining method according to the present invention;
FIG. 3A is a flowchart of Embodiment 3 of a blood pressure calibration period determining method according to the present invention;
FIG. 3B is a diagram of a relationship between a change degree and a blood pressure calibration period according to the present invention;
FIG. 4 is a flowchart of Embodiment 4 of a blood pressure calibration period determining method according to the present invention;
FIG. 5 is a schematic structural diagram of Embodiment 1 of a blood pressure calibration period determining apparatus according to the present invention; and
FIG. 6 is a schematic structural diagram of an entity of a blood pressure measurement device according to the present invention.

### DESCRIPTION OF EMBODIMENTS

To make the objectives, technical solutions, and advantages of the embodiments of the present invention clearer, the following clearly and completely describes the technical solutions in the embodiments of the present invention with reference to the accompanying drawings in the embodiments of the present invention. Apparently, the described embodiments are some but not all of the embodiments of the present invention. All other embodiments obtained by persons of ordinary skill in the art based on the embodiments of the present invention without creative efforts shall fall within the protection scope of the present invention.

The present invention may be applied to a blood pressure measurement device that measures blood pressure by using PPG. When the blood pressure is measured by using the PPG, to ensure measurement precision, a correspondence needs to be established between an actual blood pressure value of a tested person and a PPG signal characteristic, and saved as calibration information. In a subsequent measurement, the calibration information is used as a sample to calculate a blood pressure value of the tested person with reference to a current PPG signal characteristic of the tested person. However, related information that causes a change in the blood pressure of the tested person does not remain unchanged, and a change in the related information results in that the original calibration information cannot truly reflect a relationship between a PPG signal characteristic and blood pressure during an actual blood pressure measurement process. Therefore, a blood pressure measurement performed by the blood pressure measurement device needs to be calibrated. In the prior art, the following manner is mainly used: A tested person calibrates, at an interval of a fixed blood pressure calibration period, a blood pressure measurement performed by a blood pressure measurement device.

However, in the prior art, if a relatively long blood pressure calibration period (for example, calibration is performed every one to two months) is set, and related information of a user that causes a change in blood pressure within this period of time changes obviously, calibration information cannot match current related information of the user in time, resulting in an inaccurate blood pressure measurement result. In addition, if a relatively short blood pressure calibration period (for example, calibration is performed every three to four days) is set, the related information of the user does not change obviously within a relatively short period of time. Therefore, a short calibration period is of little significance for improving blood pressure measurement precision, and affects user experience.

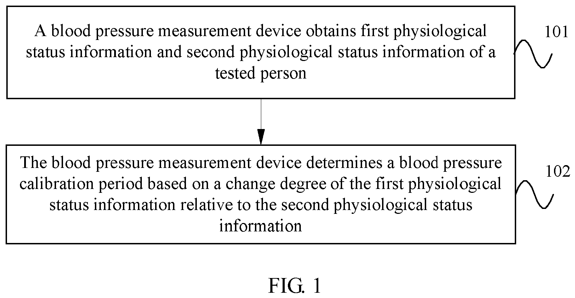

FIG. 1 is a flowchart of Embodiment 1 of a blood pressure calibration period determining method according to the present invention. As shown in FIG. 1, the method in this embodiment may include the following steps.

Step 101: A blood pressure measurement device obtains first physiological status information and second physiological status information of a tested person.

In this step, the first physiological status information is related information that causes a change in blood pressure of the tested person within a first time period after the blood pressure is calibrated, and the second physiological status information is related information that causes a change in the blood pressure of the tested person within a second time period before the blood pressure is calibrated. Specifically, the related information that causes a change in the blood pressure may be one or more types of any information that can cause a change in the blood pressure, for example, exercise intensity or exercise duration. Assuming that a calibration time is t₀, the first physiological status information may be related information that causes a change in the blood pressure of the tested person within a time period from t₀ to t₀+Δt₁ (or from t₀+1 to t₀+Δt₁+1, from t₀+2 to t₀+Δt₁+2, from t₀+3 to t₀+Δt₁+3, or the like), and the second physiological status information may be related information that causes a change in the blood pressure of the tested person within a time period from t₀-Δt₂ to t₀ (or from t₀-Δt₂-1 to t₀-1, from t₀-Δt₂-2 to t₀-2, from t₀-Δt₂-3 to t₀-3, or the like). Both Δt₁ and Δt₂ are greater than 0, and Δt₁ and Δt₂ may be the same or different (that is, a time length of the first time period may be the same as or different from a time length of the second time period). Preferably, a difference between a start time of the second time period and t₀ should be less than or equal to a preset threshold.

Step 102: The blood pressure measurement device determines a blood pressure calibration period based on a change degree of the first physiological status information relative to the second physiological status information.

In this step, the change degree of the first physiological status information relative to the second physiological status information may be a change amount of the first physiological status information relative to the second physiological status information, or may be a change rate of the first physiological status information relative to the second physiological status information. It should be noted that specific information included in the first physiological status information and specific information included in the second physiological status information should be of a same type. For example, if the specific information included in the first physiological status information is the exercise duration, the specific information included in the second physiological status information should also be the exercise duration. Assuming that both the first physiological status information and the second physiological status information include the exercise duration, the exercise duration in the first physiological status information is two hours, and the exercise duration in the second physiological status information is four hours, the change amount of the first physiological status information relative to the second physiological status information is two hours, and the change rate of the first physiological status information relative to the second physiological status information is 50%.

It should be noted that "before the blood pressure is calibrated" and "after the blood pressure is calibrated" mentioned in step 101 are based on latest blood pressure calibration performed on the blood pressure measurement device. Specifically, the latest blood pressure calibration performed on the blood pressure measurement device may be manually triggered by the user, or may be triggered by expiration of the blood pressure calibration period determined according to the method provided in the present invention.

In this embodiment, the blood pressure measurement device determines the blood pressure calibration period based on the change degree of the related information that causes a change in the blood pressure of the tested person within the first time period after the blood pressure is calibrated relative to the related information that causes a change in the blood pressure of the tested person within the second time period before the blood pressure is calibrated. Therefore, the blood pressure measurement device may adjust the blood pressure calibration period based on the related information that causes a change in the blood pressure of the tested person. Compared with a method of using a fixed blood pressure calibration period, this method implements more appropriate selection of a blood pressure calibration period, thereby improving blood pressure measurement accuracy of the blood pressure measurement device.

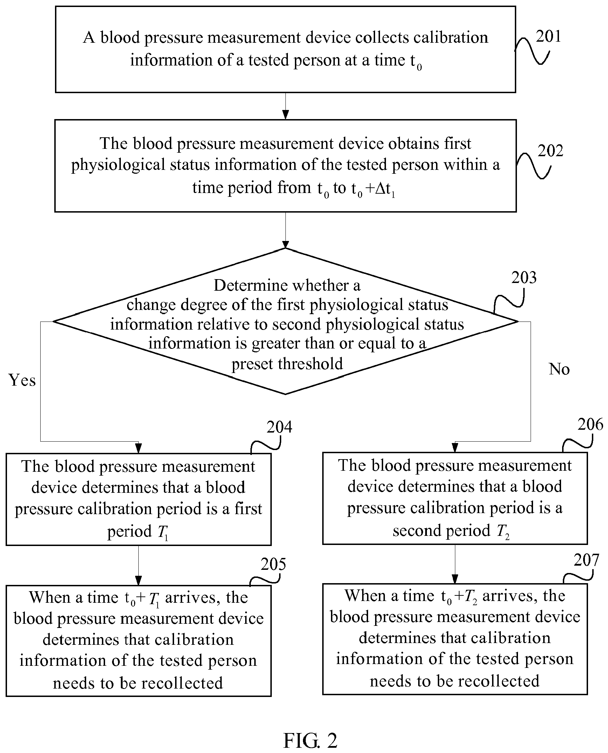

FIG. 2 is a flowchart of Embodiment 2 of a blood pressure calibration period determining method according to the present invention. According to the method in this embodiment, on the basis of the embodiment shown in FIG. 1, the following mainly describes an overall process of determining a blood pressure calibration period. As shown in FIG. 2, the method in this embodiment may include the following steps.

Step 201: A blood pressure measurement device collects calibration information of a tested person at a time t₀.

In this step, the calibration information includes an actual blood pressure value and a PPG signal. The PPG signal may be collected by using at least one of a light sensor, a pressure sensor, an acoustic sensor, a photoelectric sensor, an acceleration sensor, or a displacement sensor. The actual blood pressure value may be obtained in the following manners: (1) The blood pressure measurement device is connected to a cuff pressure pump in a near field communication manner such as Bluetooth or Wireless Fidelity (Wi-Fi, Wireless Fidelity), and the blood pressure measurement device sends an instruction to the cuff pressure pump to collect the actual blood pressure value through pressurization; (2) a micro air pump is integrated in the blood pressure measurement device, for example, a micro air pump is integrated in a watch band, to collect the actual blood pressure value through pressurization; (3) a user collects the actual blood pressure value by using a cuff blood pressure meter and inputs the actual blood pressure value into the blood pressure measurement device manually or in a near field communication manner such as Bluetooth or Wi-Fi.

Optionally, the blood pressure measurement device may obtain a PPG signal characteristic based on at least two PPG signals, or the blood pressure measurement device may obtain a signal characteristic based on a PPG signal and an electrocardiogram (ECG, Electrocardiograph) signal. The signal characteristic may be a waveform, or may be information that represents a biological signal and that is obtained after processing based on various signal processing methods, for example, at least one piece of characteristic point information. When the blood pressure measurement device obtains the signal characteristic based on the PPG signal and the electrocardiogram signal, the calibration information may further include the electrocardiogram signal. The electrocardiogram signal is collected by an electrocardiogram sensor.

It should be noted that a specific manner in which the PPG signal characteristic is obtained based on two PPG signals or the PPG signal characteristic is obtained based on the PPG signal and the electrocardiogram signal is not specifically limited in the present invention.

Step 202: The blood pressure measurement device obtains first physiological status information of the tested person within a time period from t₀ to t₀+Δt₁.

In this step, the first physiological status information is related information that causes a change in blood pressure of the tested person within the time period from t₀ to t₀+Δt₁. It should be noted that the related information that causes a change in the blood pressure of the tested person within the time period from t₀ to t₀+Δt₁ may include related information that causes a change in the blood pressure of the tested person at a time t₀ and/or a time t₀+Δt₁, or may not include related information that causes a change in the blood pressure of the tested person at a time t₀ and/or a time t₀+Δt₁.

Step 203: The blood pressure measurement device determines whether a change degree of the first physiological status information relative to second physiological status information is greater than or equal to a preset threshold.

If the change degree of the first physiological status information relative to the second physiological status information is greater than or equal to the preset threshold, step 204 is performed; or if the change degree of the first physiological status information relative to the second physiological status information is less than the preset threshold, step 206 is performed.

In this step, the second physiological status information is related information that causes a change in the blood pressure of the tested person within a time period from t₀-Δt₂ to t₀. It should be noted that the related information that causes a change in the blood pressure of the tested person within the time period from t₀-Δt₂ to t₀ may include related information that causes a change in the blood pressure of the tested person at a time t₀-Δt₂ and/or a time t₀, or may not include related information that causes a change in the blood pressure of the tested person at a time t₀-Δt₂ and/or a time t₀. Optionally, the related information includes at least one of the following information: medication information, exercise information, sleep information, or environment information.

Step 204: The blood pressure measurement device determines that a blood pressure calibration period is a first period *T*₁.

In this step, the first period is less than a preset blood pressure calibration period of the blood pressure measurement device. Optionally, the blood pressure measurement device may determine that a result of subtracting a first offset (where the first offset is greater than 0) from the preset blood pressure calibration period is the first period.

Step 205: When a time t₀ + *T*₁ arrives, the blood pressure measurement device determines that calibration information of the tested person needs to be recollected.

Step 206: The blood pressure measurement device determines that the blood pressure calibration period is a second period *T*₂.

In this step, the second period is greater than or equal to the preset blood pressure calibration period. Optionally, the blood pressure measurement device may determine that a result of adding a second offset (where the second offset is greater than or equal to 0) to the preset blood pressure calibration period is the second period.

Step 207: When a time t₀+*T*₂ arrives, the blood pressure measurement device determines that calibration information of the tested person needs to be recollected.

In this embodiment, when determining that the change degree of the first physiological status information relative to the second physiological status information is greater than or equal to the preset threshold, the blood pressure measurement device determines that the blood pressure calibration period is the first period that is less than the preset blood pressure calibration period, or when determining that the change degree of the first physiological status information relative to the second physiological status information is less than the preset threshold, the blood pressure measurement device determines that the blood pressure calibration period is the second period that is greater than or equal to the preset blood pressure calibration period. Therefore, the blood pressure measurement device may adjust the blood pressure calibration period based on the related information that causes a change in the blood pressure of the tested person, thereby improving blood pressure measurement accuracy of the blood pressure measurement device.

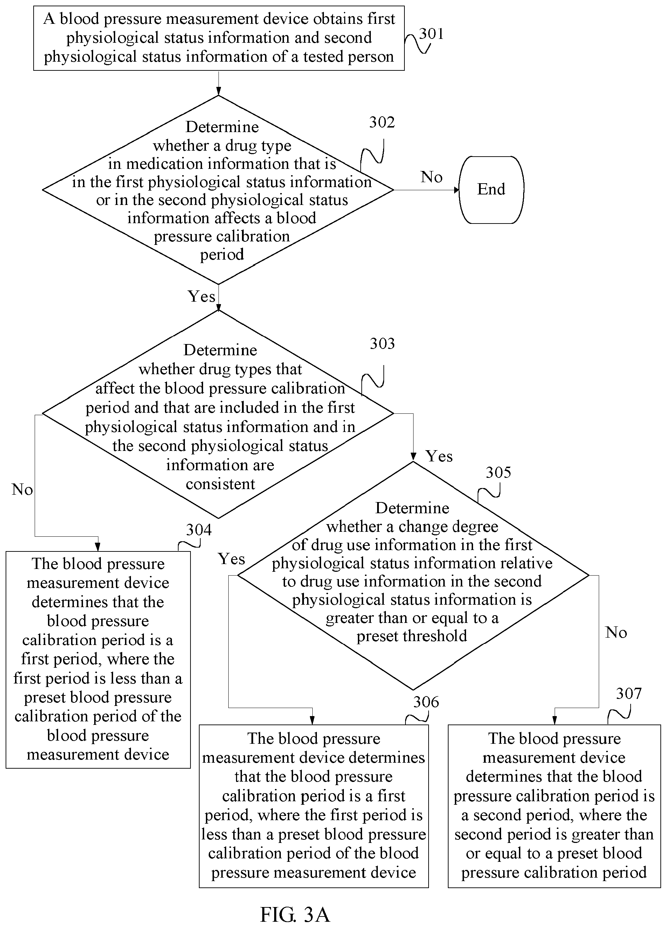

FIG. 3A is a flowchart of Embodiment 3 of a blood pressure calibration period determining method according to the present invention. According to the method in this embodiment, on the basis of the embodiment shown in FIG. 1 or FIG. 2, the following mainly describes a specific process in which a blood pressure measurement device determines a blood pressure calibration period based on a change degree of medication information that causes a change in blood pressure of a tested person within a first time period after the blood pressure is calibrated relative to medication information that causes a change in the blood pressure of the tested person within a second time period before the blood pressure is calibrated. As shown in FIG. 3A, the method in this embodiment may include the following steps.

Step 301: A blood pressure measurement device obtains first physiological status information and second physiological status information of a tested person.

In this step, the first physiological status information is the medication information that causes a change in the blood pressure of the tested person within the first time period after the blood pressure is calibrated, and the second physiological status information is the medication information that causes a change in the blood pressure of the tested person within the second time period before the blood pressure is calibrated. Optionally, the blood pressure measurement device may obtain the medication information from a medication reminding application APP; or the blood pressure measurement device may determine the medication information based on a user input. Optionally, the medication information includes a drug type and drug use information.

Step 302: The blood pressure measurement device determines whether a drug type in medication information that is in the first physiological status information or in the second physiological status information affects a blood pressure calibration period.

If the drug type in the medication information that is in the first physiological status information or in the second physiological status information affects the blood pressure calibration period, step 303 is performed; or if the drug type in the medication information that is in the first physiological status information or in the second physiological status information does not affect the blood pressure calibration period, the process ends.

In this step, the drug type may be, for example, a cardiovascular or cerebrovascular type or a vitamin type. If the drug type is the cardiovascular or cerebrovascular type (for example, an antihypertensive drug or a blood vessel softening drug), blood pressure of a blood vessel of the tested person is affected, a relationship between the blood pressure and a PPG signal characteristic is further affected, and therefore, the blood pressure calibration period is affected. If the drug type is the vitamin type, blood pressure of a blood vessel of the tested person is not affected within a period of time, and therefore, the blood pressure calibration period is not affected.

Step 303: The blood pressure measurement device determines whether drug types that affect the blood pressure calibration period and that are included in the first physiological status information and in the second physiological status information are consistent.

In this step, if the first physiological status information includes the drug type that affects the blood pressure calibration period but the second physiological status information does not include the drug type that affects the blood pressure calibration period, it is considered that the drug types that affect the blood pressure calibration period and that are included in the first physiological status information and in the second physiological status information are inconsistent. If the second physiological status information includes the drug type that affects the blood pressure calibration period but the first physiological status information does not include the drug type that affects the blood pressure calibration period, it is considered that the drug types that affect the blood pressure calibration period and that are included in the first physiological status information and in the second physiological status information are inconsistent. If both the first physiological status information and the second physiological status information include the drug type that affects the blood pressure calibration period, it is considered that the drug types that affect the blood pressure calibration period and that are included in the first physiological status information and in the second physiological status information are consistent.

In this step, when it is determined that the drug types that affect the blood pressure calibration period and that are included in the first physiological status information and in the second physiological status information are inconsistent, step 304 is then performed. For example, the tested person does not take the antihypertensive drug within the second time period (for example, greater than or equal to one week) before the blood pressure is calibrated, and takes the antihypertensive drug after the blood pressure is calibrated. After the tested person takes the antihypertensive drug, the blood pressure of the tested person changes greatly. Therefore, the blood pressure of the tested person should be re-calibrated. When it is determined that the drug types that affect the blood pressure calibration period and that are included in the first physiological status information and in the second physiological status information are consistent, step 305 is then performed, and the blood pressure calibration period is further determined based on the drug use information.

Step 304: The blood pressure measurement device determines that the blood pressure calibration period is a first period, where the first period is less than a preset blood pressure calibration period of the blood pressure measurement device.

In this step, for example, tᵢ is a current time, and t₀ is a calibration time. Then, the first period may be tᵢ-t₀. That is, re-calibration needs to be performed immediately.

It should be noted that the process ends after step 304 is performed.

Step 305: The blood pressure measurement device determines whether a change degree of drug use information in the first physiological status information relative to drug use information in the second physiological status information is greater than or equal to a preset threshold.

If the change degree of the drug use information in the first physiological status information relative to the drug use information in the second physiological status information is greater than or equal to the preset threshold, step 306 is performed; or if the change degree of the drug use information in the first physiological status information relative to the drug use information in the second physiological status information is less than the preset threshold, step 307 is performed.

In this step, optionally, the drug use information includes at least one of the following information: a drug dose, an efficacy time, or a medication regularity.

For the drug dose, if the drug types that affect the blood pressure calibration period and that are included in the first physiological status information and in the second physiological status information are consistent, but there is a relatively large difference (for example, greater than or equal to a preset threshold a) between drug doses in the first physiological status information and in the second physiological status information, the blood pressure of the tested person changes to some extent. Therefore, the blood pressure calibration period needs to be shortened from the preset blood pressure calibration period to the first period. For example, if a drug dose in the first physiological status information is relatively large and a drug dose in the second physiological status information is relatively small, the blood pressure calibration period is shortened from a preset blood pressure calibration period of 31 days to 14 days. If there is a relatively small difference between drug doses in the first physiological status information and in the second physiological status information, it is determined that the blood pressure calibration period is the second period.

For the efficacy time, if the drug types that affect the blood pressure calibration period and that are included in the first physiological status information and in the second physiological status information are consistent, for example, both are antihypertensive drug types, but there is a relatively large difference between efficacy times in the first physiological status information and in the second physiological status information (where a long-acting drug has characteristics of slow absorption, a slow onset, and a long maintain time, and a short-acting drug has characteristics of quick absorption, a quick onset, and a short maintain time), the blood pressure of the tested person changes to some extent. Therefore, the blood pressure calibration period needs to be shortened from the preset blood pressure calibration period to the first period. If there is a relatively small difference between efficacy times in the first physiological status information and in the second physiological status information (for example, the efficacy times are the same), it is determined that the blood pressure calibration period is the second period.

For the medication regularity, if there is a relatively large difference between medication regularities in the first physiological status information and in the second physiological status information (generally, higher consistency of the medication regularities indicates a smaller fluctuation in the blood pressure), for example, the first physiological status information indicates that an antihypertensive drug is taken regularly and the second physiological status information indicates that a medication regularity is reduced, the blood pressure of the tested person fluctuates. Therefore, the blood pressure calibration period needs to be shortened from the preset blood pressure calibration period to the first period. If there is a relatively small difference between medication regularities in the first physiological status information and in the second physiological status information, it is determined that the blood pressure calibration period is the second period.

It should be noted that when the blood pressure calibration period is determined by comparing the drug doses, the efficacy times, and/or the medication regularities in the first physiological status information and in the second physiological status information, the drug doses, the efficacy times, and the medication regularities may be quantified first. The efficacy time may be quantified with grading. For example, an X grade may be set for a short-acting drug, a Y grade may be set for a medium-acting drug, or a K grade may be set for a long-acting drug. If a combination of an efficacy time and a drug dose within a period of time is expressed as 5X+2Y+K (a corresponding sequence may be "5 2 1"), it may indicate that the tested person has taken five units of the short-acting drug, two units of the medium-acting drug, and one unit of the long-acting drug within the period of time. The medication regularity may be quantified on the basis of whether a drug is taken daily or not. For example, a sequence "101" is used to indicate that a drug is taken on a first day, is not taken on a second day, and is taken on a third day. Then, a change degree between the first physiological status information and the second physiological status information may be determined based on an average value (or a variance or the like) of quantified drug use information in the first physiological status information and an average value (or a variance or the like) of quantified corresponding drug use information in the second physiological status information. For example, a change degree between a medication regularity before blood pressure is calibrated and a medication regularity after the blood pressure is calibrated may be obtained by determining a change degree between an average value of a sequence "0111101000" that is obtained after the medication regularity in the first physiological status information is quantified and an average value of a sequence "0111101011" that is obtained after the medication regularity in the second physiological status information is quantified. For another example, a change degree between an efficacy time and a drug dose before blood pressure is calibrated and an efficacy time and a drug dose after the blood pressure is calibrated may be obtained by determining a change degree between an average value of a sequence "7 5 0" that is obtained after the efficacy time and the drug dose in the first physiological status information are quantified and an average value of a sequence "6 5 1" that is obtained after the efficacy time and the drug dose in the second physiological status information are quantified. Finally, the blood pressure calibration period is determined based on the change degree.

It should be noted that when the blood pressure calibration period is determined, the two types of drug use information, that is, the efficacy time and the drug dose, may be used separately, or may be used together. The following description is based on that the two types of drug use information are used together, and the efficacy time and the drug dose are collectively referred to as efficacy dose information.

Optionally, there may be a priority relationship between the drug dose and the efficacy dose information. For example, the priority relationship between the two may be: the medication regularity > the efficacy dose information. The blood pressure measurement device may successively determine, based on the priority relationship, whether change degrees of the medication regularity and the efficacy dose information are greater than or equal to a preset threshold. Preset thresholds corresponding to the medication regularity and the efficacy dose information may be the same or different. Specifically, first, the blood pressure calibration period is determined based on the change degree of the medication regularity. If it is determined that the preset blood pressure calibration period needs to be shortened, the blood pressure calibration period is shortened, and the process ends. If it is determined that the preset blood pressure calibration period does not need to be shortened, the blood pressure calibration period is further determined based on the efficacy dose information; and if it is then determined that the preset blood pressure calibration period needs to be shortened, the blood pressure calibration period is shortened, and the process ends. For example, when the blood pressure calibration period is determined based on a change rate of the medication regularity, if the change rate of the medication regularity is greater than a threshold Th1 (for example, 30%), the blood pressure calibration period is shortened to 14 days; if the change rate of the medication regularity is between a threshold Th1 and a threshold Th2 (for example, between 10% and 30%), the blood pressure calibration period is shortened to 21 days; or if the change rate of the medication regularity is less than 10%, the blood pressure calibration period is further determined based on a change rate of the efficacy dose information. When the blood pressure calibration period is determined based on the change rate of the efficacy dose information, if the change rate of the efficacy dose information is greater than a threshold Th1, the blood pressure calibration period is shortened to 14 days; if the change rate of the efficacy dose information is between a threshold Th1 and a threshold Th2, the blood pressure calibration period is shortened to 21 days; or if the change rate of the efficacy dose information is less than 10%, the blood pressure calibration period may be extended (or the blood pressure calibration period may not be updated, that is, the blood pressure calibration period is kept unchanged).

When the medication regularity and the efficacy dose information are of different priorities, weights may also be determined based on the priorities, and a change rate of the drug use information in the two pieces of physiological status information may be determined by calculating a weighted sum of the change rates of the medication regularity and the efficacy dose information. For example, it is assumed that the priority relationship between the drug dose and the efficacy dose information may be: the medication regularity > the efficacy dose information, a weight of the medication regularity is 0.6, and a weight of the efficacy dose information is 0.4. When a change rate of the medication regularity in the first physiological status information relative to the medication regularity in the second physiological status information is 28%, and a change rate of the efficacy dose information in the first physiological status information relative to the efficacy dose information in the second physiological status information is 50%, a change rate of the drug use information in the first physiological status information relative to the drug use information in the second physiological status information is 36.8%. Further, if the preset threshold is 30%, it is determined that the blood pressure calibration period is the first period that is less than the preset blood pressure calibration period. It should be noted that when the medication regularity and the efficacy dose information are of a same priority, the change rate of the drug use information in the two pieces of physiological status information may also be determined in a weighted manner, where weights are the same.

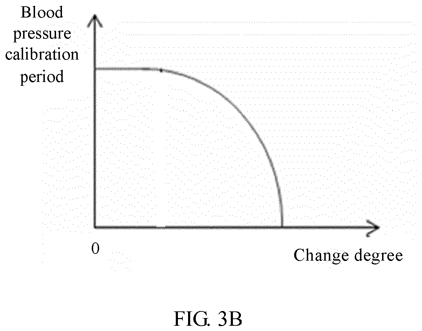

It should be noted that when the blood pressure calibration period is determined based on the change degree of the drug use information, the blood pressure calibration period may be determined based on a relationship that a larger change degree indicates a shorter blood pressure calibration period. The relationship, for example, may be shown in FIG. 3B. It should be noted that FIG. 3B is merely an example, and a relationship between the change degree and the blood pressure calibration period may also be another monotonically decreasing relationship that a larger change degree indicates a shorter blood pressure calibration period, for example, a linear relationship.

Step 306: The blood pressure measurement device determines that the blood pressure calibration period is a first period, where the first period is less than a preset blood pressure calibration period of the blood pressure measurement device.

For example, if the preset blood pressure calibration period is 31 days, the first period may be 14 days.

It should be noted that the process ends after step 306 is performed.

Step 307: The blood pressure measurement device determines that the blood pressure calibration period is a second period, where the second period is greater than or equal to the preset blood pressure calibration period.

For example, if the preset blood pressure calibration period is 31 days, the first period may be 31 days or 40 days.

In this embodiment, the blood pressure measurement device determines the blood pressure calibration period based on the medication information that causes a change in the blood pressure of the tested person within the first time period after the blood pressure is calibrated and the medication information that causes a change in the blood pressure of the tested person within the second time period before the blood pressure is calibrated. Therefore, the blood pressure measurement device may adjust the blood pressure calibration period based on the medication information that causes a change in the blood pressure, thereby improving blood pressure measurement accuracy of the blood pressure measurement device.

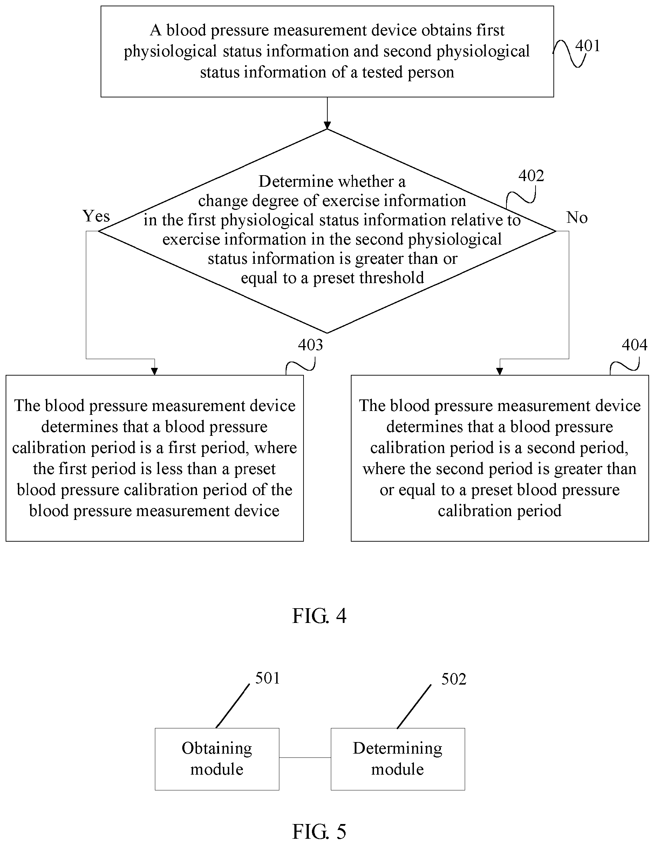

FIG. 4 is a flowchart of Embodiment 4 of a blood pressure calibration period determining method according to the present invention. According to the method in this embodiment, on the basis of the embodiment shown in FIG. 1 or FIG. 2, the following mainly describes a process in which a blood pressure calibration period is determined based on a change degree of exercise information that causes a change in blood pressure of a tested person within a first time period after the blood pressure is calibrated relative to exercise information that causes a change in the blood pressure of the tested person within a second time period before the blood pressure is calibrated. As shown in FIG. 4, the method in this embodiment may include the following steps.

Step 401: A blood pressure measurement device obtains first physiological status information and second physiological status information of a tested person.

In this step, the first physiological status information includes the exercise information that causes a change in the blood pressure of the tested person within the first time period after the blood pressure is calibrated, and the second physiological status information includes the exercise information that causes a change in the blood pressure of the tested person within the second time period before the blood pressure is calibrated. Optionally, the blood pressure measurement device may determine the exercise information based on information output by an acceleration sensor and/or a gyro sensor that are/is disposed on the blood pressure measurement device; or the blood pressure measurement device may determine the exercise information based on a user input.

Step 402: The blood pressure measurement device determines whether a change degree of exercise information in the first physiological status information relative to exercise information in the second physiological status information is greater than or equal to a preset threshold.

If the change degree of the exercise information in the first physiological status information relative to the exercise information in the second physiological status information is greater than or equal to the preset threshold, step 403 is performed; or if the change degree of the exercise information in the first physiological status information relative to the exercise information in the second physiological status information is less than the preset threshold, step 404 is performed.

In this step, optionally, the exercise information includes at least one of the following information: exercise intensity, exercise duration, or an exercise regularity. Physical exercise of the tested person may cause a change in the blood pressure of the tested person, for example, an increase in myocardial contractility, and a relationship between a PPG signal characteristic and the blood pressure is affected. Therefore, the exercise information affects the blood pressure, and the blood pressure calibration period may be determined based on the exercise information.

For the exercise regularity, if there is a relatively large difference (for example, greater than or equal to a preset threshold b) between an exercise regularity within the first time period after the blood pressure is calibrated and an exercise regularity within the second time period before the blood pressure is calibrated, the blood pressure calibration period needs to be shortened from a preset blood pressure calibration period to a first period. For example, if a user does not take regular exercise within the second time period before the blood pressure is calibrated but takes long-term regular exercise within a period of time after the blood pressure is calibrated, the blood pressure calibration period may be shortened from a preset blood pressure calibration period of 31 days to 14 days. If there is a relatively small difference between an exercise regularity within the first time period after the blood pressure is calibrated and an exercise regularity within the second time period before the blood pressure is calibrated, it is determined that the blood pressure calibration period is a second period.

For the exercise duration, if there is a relatively large difference between exercise duration within the first time period after the blood pressure is calibrated and exercise duration within the second time period before the blood pressure is calibrated, the blood pressure calibration period needs to be shortened from a preset blood pressure calibration period to a first period. If there is a relatively small difference between exercise duration within the first time period after the blood pressure is calibrated and exercise duration within the second time period before the blood pressure is calibrated, it is determined that the blood pressure calibration period is a second period.

For the exercise intensity, if there is a relatively large difference between exercise duration within the first time period after the blood pressure is calibrated and exercise intensity within the second time period before the blood pressure is calibrated, the blood pressure calibration period needs to be shortened from a preset blood pressure calibration period to a first period. If there is a relatively small difference between exercise intensity within the first time period after the blood pressure is calibrated and exercise intensity within the second time period before the blood pressure is calibrated, it is determined that the blood pressure calibration period is a second period.

It should be noted that when the blood pressure calibration period is determined by comparing the exercise regularities, the exercise duration, and/or the exercise intensity in the first physiological status information and in the second physiological status information, the exercise regularities, the exercise duration, and/or the exercise intensity may be separately quantified first. The exercise intensity may be quantified with grading, for example, quantified as an x grade, a y grade, or a c grade. If a combination of exercise intensity and exercise duration within a period of time is expressed as 5x+2y+k, it may indicate that the tested person has taken five hours of x-grade exercise, two hours of y-grade exercise, and one hour of c-grade exercise within the period of time. The exercise regularity may be quantified on the basis of whether exercise is taken daily or not. For example, a sequence "101" is used to indicate that exercise is taken on a first day, is not taken on a second day, and is taken on a third day. Then, a change degree between the first physiological status information and the second physiological status information is determined based on an average value (or a variance or the like) of quantified exercise information in the first physiological status information and an average value (or a variance or the like) of quantified corresponding exercise information in the second physiological status information, and the blood pressure calibration period is determined based on the change degree. It should be noted that a manner, in this embodiment, in which a change degree between the exercise information before the blood pressure is calibrated and the exercise information after the blood pressure is calibrated is determined based on the quantified exercise information, is similar to the foregoing manner in which a change degree between the drug use information before the blood pressure is calibrated and the drug use information after the blood pressure is calibrated is determined based on the quantified drug use information, and details are not described herein.

It should be noted that in this embodiment, the blood pressure calibration period may also be determined based on a priority relationship between the exercise information. A specific process is similar to the foregoing manner in which the blood pressure calibration period is determined based on the priority relationship between the drug use information, and details are not described herein.

It should be noted that when the blood pressure calibration period is determined based on the change degree of the exercise information, the blood pressure calibration period may be determined based on a relationship that a larger change degree indicates a shorter blood pressure calibration period.

Step 403: The blood pressure measurement device determines that the blood pressure calibration period is a first period, where the first period is less than a preset blood pressure calibration period of the blood pressure measurement device.

It should be noted that step 403 is similar to step 306, and details are not described herein.

Step 404: The blood pressure measurement device determines that the blood pressure calibration period is a second period, where the second period is greater than or equal to the preset blood pressure calibration period.

It should be noted that step 404 is similar to step 307, and details are not described herein.

In this embodiment, the blood pressure measurement device determines the blood pressure calibration period based on the exercise information that causes a change in the blood pressure of the tested person within the first time period after the blood pressure is calibrated and the exercise information that causes a change in the blood pressure of the tested person within the second time period before the blood pressure is calibrated. Therefore, the blood pressure measurement device may adjust the blood pressure calibration period based on the exercise information that causes a change in the blood pressure, thereby improving blood pressure measurement accuracy of the blood pressure measurement device.

Embodiment 5 of a blood pressure calibration period determining method

Optionally, on the basis of Embodiment 3 or Embodiment 4 of the blood pressure calibration period determining method according to the present invention, the related information may further include environment information and/or sleep information.

Optionally, the environment information includes human body environment information and/or external environment information. Optionally, the external environment information includes at least one of the following information: an ambient temperature, an altitude, contact pressure between a measured part and a sensor of the blood pressure measurement device, or ambient light intensity. Optionally, the human body environment information may include a human body temperature. Optionally, the blood pressure measurement device may determine the sleep information based on information output by an acceleration sensor, a gyro sensor, and a photo plethysmo graphy PPG sensor that are disposed on the blood pressure measurement device. Optionally, the sleep information includes a sleep time and/or sleep quality.

A change in the sleep information and in the environment information causes a change in blood pressure of a tested person. Therefore, a blood pressure calibration period may be determined based on a change degree of sleep information and/or environment information within a first time period after the blood pressure is calibrated relative to sleep information and/or environment information within a second time period before the blood pressure is calibrated.

For the sleep time, if there is a relatively large difference between a sleep time within the first time period after the blood pressure is calibrated and a sleep time within the second time period before the blood pressure is calibrated, the blood pressure calibration period needs to be shortened from a preset blood pressure calibration period to a first period. If there is a relatively small difference between a sleep time within the first time period after the blood pressure is calibrated and a sleep time within the second time period before the blood pressure is calibrated, it is determined that the blood pressure calibration period is a second period.

For the sleep quality, if there is a relatively large difference between sleep quality within the first time period after the blood pressure is calibrated and sleep quality within the second time period before the blood pressure is calibrated, the blood pressure calibration period needs to be shortened from a preset blood pressure calibration period to a first period. If there is a relatively small difference between sleep quality within the first time period after the blood pressure is calibrated and sleep quality within the second time period before the blood pressure is calibrated, it is determined that the blood pressure calibration period is a second period.

It should be noted that when the blood pressure calibration period is determined by comparing the sleep times and/or the sleep quality in first physiological status information and in second physiological status information, the sleep times and/or the sleep quality may be quantified first. The sleep quality may be quantified with grading, for example, quantified as an L grade that is corresponding to a deep sleep period and an M grade that is corresponding to a light sleep period and another period. If a combination of sleep quality and a sleep time on a day is expressed as 3A+5B, it may indicate that the tested person has undergone a deep sleep period of three hours and another sleep period, except the deep sleep period, of five hours on the day. Then, a change degree between the first physiological status information and the second physiological status information is determined based on an average value (or a variance or the like) of quantified sleep information in the first physiological status information and an average value (or a variance or the like) of quantified corresponding sleep information in the second physiological status information, and the blood pressure calibration period is determined based on the change degree. It should be noted that a manner, in this embodiment, in which a change degree between the exercise information before the blood pressure is calibrated and the exercise information after the blood pressure is calibrated is determined based on the quantified sleep information, is similar to the foregoing manner in which a change degree between the drug use information before the blood pressure is calibrated and the drug use information after the blood pressure is calibrated is determined based on the quantified drug use information, and details are not described herein.

It should be noted that a specific process, in this embodiment, in which the blood pressure calibration period is determined based on the environment information is similar to the specific process, shown in FIG. 4, in which the blood pressure calibration period is determined based on the exercise information, and details are not described herein.

In this embodiment, the blood pressure measurement device determines the blood pressure calibration period based on environment information and/or sleep information that cause/causes a change in the blood pressure of the tested person within the first time period after the blood pressure is calibrated and environment information and/or sleep information that cause/causes a change in the blood pressure of the tested person within the second time period before the blood pressure is calibrated. Therefore, the blood pressure measurement device may adjust the blood pressure calibration period based on the environment information and/or the sleep information that cause/causes a change in the blood pressure, thereby improving blood pressure measurement accuracy of the blood pressure measurement device.

Optionally, a priority of the medication information is higher than priorities of the exercise information, the sleep information, and the environment information. Further, optionally, there may also be a priority relationship among the exercise information, the sleep information, and the environment information.

It should be noted that when the related information includes the medication information, it may be determined, preferentially based on the medication information, whether the blood pressure calibration period needs to be shortened. If it is determined, based on the medication information, that the blood pressure calibration period does not need to be shortened, the blood pressure calibration period is further determined based on the exercise information, the sleep information, and the environment information.

When the blood pressure calibration period is determined based on the exercise information, the sleep information, and the environment information, the blood pressure calibration period may be determined successively based on the priority relationship among the exercise information, the sleep information, and the environment information. Specifically, assuming that the priorities of the exercise information, the sleep information, and the environment information are in descending order, it may be determined, first based on the exercise information, whether the blood pressure calibration period needs to be shortened. If it is determined that the blood pressure calibration period needs to be shortened, the blood pressure calibration period is shortened, and the process ends. If it is determined that the blood pressure calibration period does not need to be shortened, the blood pressure calibration period is further determined based on the sleep information; and if it is then determined that the blood pressure calibration period needs to be shortened, the blood pressure calibration period is shortened, and the process ends, or if it is determined that the blood pressure calibration period does not need to be shortened, the blood pressure calibration period is further determined based on the environment information.

When the blood pressure calibration period is determined based on the exercise information, the sleep information, and the environment information, weights may also be determined based on the priority relationship among the exercise information, the sleep information, and the environment information, and the blood pressure calibration period may be determined in a weighted manner.

FIG. 5 is a schematic structural diagram of Embodiment 1 of a blood pressure calibration period determining apparatus according to the present invention. The blood pressure calibration period determining apparatus may become a part or all of a blood pressure measurement device by using software, hardware, or a combination of software and hardware. As shown in FIG. 5, the apparatus in this embodiment may include an obtaining module 501 and a determining module 502. The obtaining module 501 is configured to obtain first physiological status information and second physiological status information of a tested person. The first physiological status information is related information that causes a change in blood pressure of the tested person within a first time period after the blood pressure is calibrated. The second physiological status information is related information that causes a change in the blood pressure of the tested person within a second time period before the blood pressure is calibrated. The determining module 502 is configured to determine a blood pressure calibration period based on a change degree of the first physiological status information relative to the second physiological status information.

The apparatus in this embodiment may be configured to execute the technical solution of the method embodiment shown in FIG. 1, implementation principles and technical effects thereof are similar, and details are not described herein.

Embodiment 2 of a blood pressure calibration period determining apparatus

Optionally, on the basis of Embodiment 1 of the blood pressure calibration period determining apparatus according to the present invention, the determining module 502 is specifically configured to: determine whether the change degree of the first physiological status information relative to the second physiological status information is greater than or equal to a preset threshold; and if the change degree is greater than or equal to the preset threshold, determine that the blood pressure calibration period is a first period, where the first period is less than a preset blood pressure calibration period of the blood pressure measurement device.

Optionally, the determining module 502 is further configured to: if the change degree is less than the preset threshold, determine that the blood pressure calibration period is a second period, where the second period is greater than or equal to the preset blood pressure calibration period.

Optionally, the related information includes at least one of the following information: medication information, exercise information, sleep information, or environment information.

The apparatus in this embodiment may be configured to execute the technical solution of the method embodiment shown in FIG. 2, implementation principles and technical effects thereof are similar, and details are not described herein.

Embodiment 3 of a blood pressure calibration period determining apparatus

Optionally, on the basis of Embodiment 1 or Embodiment 2 of the blood pressure calibration period determining apparatus according to the present invention, the medication information includes a drug type and drug use information, and the drug use information includes at least one of the following information: a drug dose, an efficacy time, or a medication regularity.

Optionally, that the obtaining module 501 obtains the medication information of the tested person specifically includes: obtaining the medication information from a medication reminding application; or determining the medication information based on a user input.

The apparatus in this embodiment may be configured to execute the technical solution of the method embodiment shown in FIG. 3A, implementation principles and technical effects thereof are similar, and details are not described herein.

Embodiment 4 of a blood pressure calibration period determining apparatus

Optionally, on the basis of Embodiment 1 or Embodiment 2 of the blood pressure calibration period determining apparatus according to the present invention, the exercise information includes at least one of the following information: exercise intensity, exercise duration, or an exercise regularity.

Optionally, that the obtaining module 501 obtains the exercise information of the tested person specifically includes: determining the exercise information based on information output by an acceleration sensor and/or a gyro sensor that are/is disposed on the apparatus; or determining the exercise information based on a user input.

The apparatus in this embodiment may be configured to execute the technical solution of the method embodiment shown in FIG. 4, implementation principles and technical effects thereof are similar, and details are not described herein.

Embodiment 5 of a blood pressure calibration period determining apparatus

Optionally, on the basis of Embodiment 1 or Embodiment 2 of the blood pressure calibration period determining apparatus according to the present invention, the sleep information includes a sleep time and/or sleep quality.

Optionally, that the obtaining module 501 obtains the sleep information of the tested person specifically includes: determining the sleep information based on information output by an acceleration sensor, a gyro sensor, and a photo plethysmo graphy PPG sensor that are disposed on the apparatus.

Optionally, the environment information includes human body environment information and/or external environment information.

Optionally, the external environment information includes at least one of the following information: an ambient temperature, an altitude, contact pressure between a measured part and a sensor of the blood pressure measurement device, or ambient light intensity.

Optionally, a priority of the medication information is higher than priorities of the exercise information, the sleep information, and the environment information.

The apparatus in this embodiment may be configured to execute the technical solution of Embodiment 5 of the blood pressure calibration period determining method, implementation principles and technical effects thereof are similar, and details are not described herein.

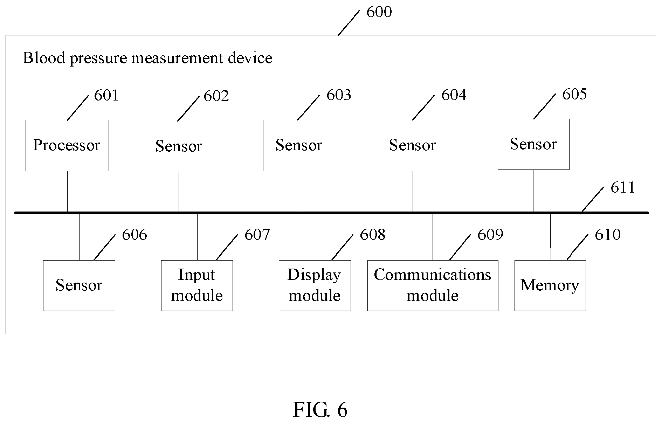

FIG. 6 is a schematic structural diagram of an entity of a blood pressure measurement device according to the present invention. As shown in FIG. 6, a blood pressure measurement device 600 in this embodiment may include a processor 601, a sensor 602, a sensor 603, a sensor 604, a sensor 605, a sensor 606, an input module 607, a display module 608, a communications module 609, a memory 610, and a bus 611. The blood pressure measurement device 600 is not limited to the components shown in FIG. 6, and therefore, more or fewer components may be used to implement the blood pressure measurement device 600.

The blood pressure measurement device 600 includes more than one sensor, for example, the sensor 602, the sensor 603, the sensor 604, the sensor 605, and the sensor 606 in FIG. 6. The sensor 602 is an acceleration sensor, the sensor 603 is a gyro sensor, the sensor 604 is a photo plethysmo graphy (PPG) sensor, the sensor 605 is a pressure sensor, and the sensor 606 is a light sensor. The acceleration sensor 602 is configured to measure linear acceleration of the blood pressure measurement device 600. The gyro sensor 603 is configured to measure an angular velocity of the blood pressure measurement device 600. The PPG sensor 604 records light change induction and generates a signal by using a principle that a light sensor element absorbs light energy. The pressure sensor 605 is configured to measure contact pressure between a measured part and the blood pressure measurement device 600. The light sensor 606 is configured to measure ambient light intensity.

The blood pressure measurement device 600 further includes the input module 607. The input module 607 may receive a command or data from a user, and transfer the command or the data to the processor 601 or the memory 610 by using the bus 611. For example, the input module 607 may include a touchpad (touch panel), a keyboard, or an ultrasonic input apparatus. The touchpad may recognize a touch input in at least one of a capacitive manner, a pressure-sensitive manner, an infrared manner, or an ultrasonic manner. The touchpad may further include a controller. A capacitive touchpad may recognize not only a direct touch but also proximity. The touchpad may further include a tactile layer (tactile layer). In this case, the touchpad may provide a tactile response for the user. The keyboard may include a keyboard or a touch key. The ultrasonic input apparatus is an apparatus that may sense a microsonic wave on an electronic apparatus by using an ultrasonic signal generation pen, so as to confirm data, and may be configured to implement wireless identification.

The blood pressure measurement device 600 further includes the display module 608. The display module 608 may display a graph, an image, or data to the user. For example, the display module 608 may include a panel. For example, the panel may be an LCD (liquid-crystal display, liquid crystal display), an LED (light emitting diode display, light emitting diode panel), or an AMOLED (active-matrix organic light-emitting diode, active matrix/organic light emitting diode). In addition, the panel may be constructed to be flexible (flexible), transparent (transparent), or wearable (wearable). The panel may also constitute a module together with the touchpad. In addition, the display module 608 may further include a control circuit that is used for a control panel.

The blood pressure measurement device 600 further includes the communications module 609, so that the blood pressure measurement device 600 may communicate bidirectionally with one or more other electronic apparatuses or servers (not shown) by using any appropriate communications protocol. The communications module 609 may support a near field communications protocol such as Wi-Fi (wireless fidelity, Wireless Fidelity), Bluetooth (BT: Bluetooth), or near field communication (NFC: near field communication), or a communications protocol used by a network such as the Internet (Internet), a local area network (LAN: local area network), a wide area network (WAN: wide area network), a telecommunications network (telecommunications network), a cellular network (cellular network), or a satellite network (satellite network). The communications module 609 may further include a circuit that enables the blood pressure measurement device 600 to be coupled with another device (for example, a computer) and communicate bidirectionally with the another device in a wired or wireless manner.

The bus 611 may be a circuit that connects composition elements (for example, the processor 601, the memory 610, the sensor 602, the sensor 603, the sensor 604, the sensor 605, the sensor 606, the input module 607, and the display module 608) included in the blood pressure measurement device 600 to each other and that implements communication between the composition elements.

The processor 601 is configured to execute an instruction (for example, an instruction obtained from the input module 607) and perform interrupt processing, timing, and another function. In addition, the processor 601 may further include a graphics processing unit (graphic processing unit).

The memory 610 may store an instruction or data that is received by the processor 601 or another composition element (for example, the input module 607, the display module 608, or the communications module 609) or that is generated by the processor 601 or another composition element. In this case, the memory 610 may include an internal buffer and an external buffer.

When the blood pressure measurement device 600 is operating, the processor 601 executes the instruction stored in the memory 610 to perform the following operations:
obtaining first physiological status information and second physiological status information of a tested person, where the first physiological status information is related information that causes a change in blood pressure of the tested person within a first time period after the blood pressure is calibrated, and the second physiological status information is related information that causes a change in the blood pressure of the tested person within a second time period before the blood pressure is calibrated; and
determining a blood pressure calibration period based on a change degree of the first physiological status information relative to the second physiological status information.

Optionally, that the processor 601 determines a blood pressure calibration period based on a change degree of the first physiological status information relative to the second physiological status information specifically includes:
determining whether the change degree of the first physiological status information relative to the second physiological status information is greater than or equal to a preset threshold; and
if the change degree is greater than or equal to the preset threshold, determining that the blood pressure calibration period is a first period, where the first period is less than a preset blood pressure calibration period of the blood pressure measurement device.

Optionally, the processor 601 is further configured to:
if the change degree is less than the preset threshold, determine that the blood pressure calibration period is a second period, where the second period is greater than or equal to the preset blood pressure calibration period.

Optionally, the related information includes at least one of the following information: medication information, exercise information, sleep information, or environment information.

Optionally, the medication information includes a drug type and drug use information, and the drug use information includes at least one of the following information: a drug dose, an efficacy time, or a medication regularity.

Optionally, that the processor 601 obtains the medication information of the tested person specifically includes: obtaining the medication information from a medication reminding application APP by using the communications module 609; or determining the medication information based on a user input obtained by using the input module 607.

Optionally, the exercise information includes at least one of the following information: exercise intensity, exercise duration, or an exercise regularity.

Optionally, that the processor 601 obtains the exercise information of the tested person specifically includes: determining the exercise information based on information output by the acceleration sensor 602 and/or the gyro sensor 603; or determining the exercise information based on a user input obtained by using the input module 607.

Optionally, the sleep information includes a sleep time and/or sleep quality.

Optionally, that the processor 601 obtains the sleep information of the tested person specifically includes: determining the sleep information based on information output by the acceleration sensor 602, the gyro sensor 603, and the photo plethysmo graphy PPG sensor 604.

Optionally, the environment information includes human body environment information and/or external environment information.

Optionally, the external environment information includes at least one of the following information: an ambient temperature, an altitude, contact pressure between a measured part and a sensor of the blood pressure measurement device, or ambient light intensity.

Optionally, a priority of the medication information is higher than priorities of the exercise information, the sleep information, and the environment information.

The apparatus in this embodiment may be configured to execute the technical solution of any method embodiment of Embodiment 1 to Embodiment 5 of the blood pressure calibration period determining method, implementation principles and technical effects thereof are similar, and details are not described herein.

Persons of ordinary skill in the art may understand that all or some of the steps of the foregoing method embodiments may be implemented by a program instructing relevant hardware. The program may be stored in a computer-readable storage medium. When the program runs, the steps of the foregoing method embodiments are performed. The foregoing storage medium includes: any medium that can store program code, such as a ROM, a RAM, a magnetic disk, or an optical disc.

Finally, it should be noted that the foregoing embodiments are merely intended for describing the technical solutions of the present invention, but not for limiting the present invention as defined in the claims. Although the present invention is described in detail with reference to the foregoing embodiments, persons of ordinary skill in the art should understand that they may still make modifications to the technical solutions described in the foregoing embodiments, without departing from the scope of the technical solutions of the embodiments of the present invention as defined in the claims.

## Claims

1. A blood pressure calibration period determining method, comprising:
obtaining (101), by a blood pressure measurement device (600), first physiological status information and second physiological status information of a tested person, wherein the first physiological status information is related information that causes a change in blood pressure of the tested person within a first time period after the blood pressure is calibrated, and the second physiological status information is related information that causes a change in the blood pressure of the tested person within a second time period before the blood pressure is calibrated; and
determining (102), by the blood pressure measurement device (600), a blood pressure calibration period based on a change degree of the first physiological status information relative to the second physiological status information,
wherein the related information comprises exercise information which comprises at least one of the following information: exercise intensity, exercise duration, or an exercise regularity,
wherein obtaining (101), by the blood pressure measurement device, the exercise information of the tested person comprises:
determining, by the blood pressure measurement device (600), the exercise information based on information output by an acceleration sensor (602) and/or a gyro sensor (603) that are/is disposed on the blood pressure measurement device (600), and
the blood pressure measurement device (600) further includes a communications module (609), so that the blood pressure measurement device (600) communicates bidirectionally with one or more other electronic apparatuses or servers, the communications module (609) supports a near field communications protocol, or near field communication, or a communications protocol used by a network.

2. The method according to claim 1, wherein the determining (101), by the blood pressure measurement device (600), the blood pressure calibration period based on the change degree of the first physiological status information relative to the second physiological status information comprises:
Determining (203), by the blood pressure measurement device (600), whether the change degree of the first physiological status information relative to the second physiological status information is greater than or equal to a preset threshold; and
if the change degree is greater than or equal to the preset threshold, determining (403) that the blood pressure calibration period is a first period, wherein the first period is less than a preset blood pressure calibration period of the blood pressure measurement device.

3. The method according to claim 2, wherein the method further comprises:
if the change degree is less than the preset threshold, determining (404) that the blood pressure calibration period is a second period, wherein the second period is greater than or equal to the preset blood pressure calibration period.

4. The method according to any one of claims 1 to 3, wherein the related information also comprises at least one of the following information:
medication information, sleep information, or environment information.

5. The method according to claim 4, wherein the related information comprises the medication information which comprises a drug type and drug use information, and the drug use information comprises at least one of the following information: a drug dose, an efficacy time, or a medication regularity.

6. The method according to claim 5, wherein obtaining, by the blood pressure measurement device (600), the medication information of the tested person comprises:
obtaining, by the blood pressure measurement device (600), the medication information from a medication reminding application; or
determining, by the blood pressure measurement device (600), the medication information based on a user input.

7. The method according to any of claims 1 to 6, wherein obtaining, by the blood pressure measurement device (600), the exercise information of the tested person also comprises:
determining, by the blood pressure measurement device (600), the exercise information also based on a user input.

8. The method according to claim 4, wherein the related information comprises the sleep information which comprises a sleep time and/or sleep quality.

9. The method according to claim 8, wherein obtaining, by the blood pressure measurement device (600), the sleep information of the tested person comprises:
determining, by the blood pressure measurement device (600), the sleep information based on information output by the acceleration sensor (602), the gyro sensor (603), and a photo plethysmo graphy, PPG, sensor (604) that are disposed on the blood pressure measurement device (600).

10. A blood pressure measurement device (600), comprising a processor (601) and a memory (610), wherein
wherein the processor (601) executes an instruction stored in the memory (610) to perform the following operations:
obtaining first physiological status information and second physiological status information of a tested person, wherein the first physiological status information is related information that causes a change in blood pressure of the tested person within a first time period after the blood pressure is calibrated, and the second physiological status information is related information that causes a change in the blood pressure of the tested person within a second time period before the blood pressure is calibrated; and
determining a blood pressure calibration period based on a change degree of the first physiological status information relative to the second physiological status information,
wherein the related information comprises exercise information, which comprises at least one of the following information: exercise intensity, exercise duration, or an exercise regularity,
wherein obtaining, by the blood pressure measurement device (600), the exercise information of the tested person comprises:
determining, by the blood pressure measurement device (600), the exercise information based on information output by an acceleration sensor (602) and/or a gyro sensor (603) that are/is disposed on the blood pressure measurement device (600), and
the blood pressure measurement device (600) further includes a communications module (609), so that the blood pressure measurement device (600) is configured to communicate bidirectionally with one or more other electronic apparatuses or servers, the communications module (609) is configured to support a near field communications protocol, or near field communication, or a communications protocol used by a network.

11. The blood pressure measurement device (600) according to claim 10, wherein the determining the blood pressure calibration period based on the change degree of the first physiological status information relative to the second physiological status information comprises:
determining whether the change degree of the first physiological status information relative to the second physiological status information is greater than or equal to a preset threshold; and
if the change degree is greater than or equal to the preset threshold, determining that the blood pressure calibration period is a first period, wherein the first period is less than a preset blood pressure calibration period of the blood pressure measurement device (600).

12. The blood pressure measurement device (600) according to claim 10, wherein the operations comprise:
if the change degree is less than the preset threshold, determine that the blood pressure calibration period is a second period, wherein the second period is greater than or equal to the preset blood pressure calibration period.

13. The blood pressure measurement device (600) according to any one of claims 10 to 12, wherein the related information also comprises at least one of the following information:
medication information, sleep information, or environment information.

14. The blood pressure measurement device (600) according to claim 13, wherein the related information comprises the medication information which comprises a drug type and drug use information, and the drug use information comprises at least one of the following information: a drug dose, an efficacy time, or a medication regularity.

## Patentansprüche

1. Verfahren zum Bestimmen eines Blutdruckkalibrierungszeitraums, das Folgendes umfasst:
Erhalten (101), durch eine Blutdruckmessvorrichtung (600), von ersten physiologischen Statusinformationen und zweiten physiologischen Statusinformationen einer getesteten Person, wobei die ersten physiologischen Statusinformationen verwandte Informationen sind, die eine Änderung des Blutdrucks der getesteten Person innerhalb eines ersten Zeitraums bewirken, nachdem der Blutdruck kalibriert wird, und die zweiten physiologischen Statusinformationen verwandte Informationen sind, die eine Änderung des Blutdrucks der getesteten Person innerhalb eines zweiten Zeitraums bewirken, bevor der Blutdruck kalibriert wird; und
Bestimmen (102), durch die Blutdruckmessvorrichtung (600), eines Blutdruckkalibrierungszeitraums basierend auf einem Änderungsgrad der ersten physiologischen Statusinformationen relativ zu den zweiten physiologischen Statusinformationen,
wobei die verwandten Informationen Bewegungsinformationen umfassen, die die folgenden Informationen umfassen: Bewegungsintensität, Bewegungsdauer und/oder eine Bewegungsregelmäßigkeit,
wobei das Erhalten (101), durch die Blutdruckmessvorrichtung, der Bewegungsinformationen der getesteten Person Folgendes umfasst:
Bestimmen, durch die Blutdruckmessvorrichtung (600), der Bewegungsinformationen basierend auf Informationen, die durch einen Beschleunigungssensor (602) und/oder einen Kreiselsensor (603) ausgegeben werden, der/die an der Blutdruckmessvorrichtung (600) angeordnet ist/sind, und
die Blutdruckmessvorrichtung (600) ferner ein Kommunikationsmodul (609) beinhaltet, so dass die Blutdruckmessvorrichtung (600) mit einer oder mehreren anderen elektronischen Einrichtungen oder einem oder mehreren anderen Servern bidirektional kommuniziert, wobei das Kommunikationsmodul (609) ein Nahfeldkommunikationsprotokoll oder eine Nahfeldkommunikation oder ein Kommunikationsprotokoll unterstützt, das durch ein Netzwerk verwendet wird.

2. Verfahren nach Anspruch 1, wobei das Bestimmen (101), durch die Blutdruckmessvorrichtung (600), des Blutdruckkalibrierungszeitraums basierend auf dem Änderungsgrad der ersten physiologischen Statusinformationen relativ zu den zweiten physiologischen Statusinformationen Folgendes umfasst:
Bestimmen (203), durch die Blutdruckmessvorrichtung (600), ob der Änderungsgrad der ersten physiologischen Statusinformationen relativ zu den zweiten physiologischen Statusinformationen größer als oder gleich einem voreingestellten Schwellenwert ist; und
falls der Änderungsgrad größer als oder gleich dem voreingestellten Schwellenwert ist, Bestimmen (403), dass der Blutdruckkalibrierungszeitraum ein erster Zeitraum ist, wobei der erste Zeitraum kleiner als ein voreingestellter Blutdruckkalibrierungszeitraum der Blutdruckmessvorrichtung ist.

3. Verfahren nach Anspruch 2, wobei das Verfahren ferner Folgendes umfasst:
falls der Änderungsgrad kleiner als der voreingestellte Schwellenwert ist, Bestimmen (404), dass der Blutdruckkalibrierungszeitraum ein zweiter Zeitraum ist, wobei der zweite Zeitraum größer als oder gleich dem voreingestellten Blutdruckkalibrierungszeitraum ist.

4. Verfahren nach einem der Ansprüche 1 bis 3, wobei die verwandten Informationen ebenso folgende Informationen umfassen:
Medikationsinformationen, Schlafinformationen und/oder Umgebungsinformationen.

5. Verfahren nach Anspruch 4, wobei die verwandten Informationen die Medikationsinformationen umfassen, die eine Arzneimittelart und Arzneimittelkonsuminformationen umfassen, und die Arzneimittelkonsuminformationen folgende Informationen umfassen: eine Arzneimitteldosis, eine Wirksamkeitszeit und/oder eine Medikationsregelmäßigkeit.

6. Verfahren nach Anspruch 5, wobei das Erhalten, durch die Blutdruckmessvorrichtung (600), der Medikationsinformationen der getesteten Person Folgendes umfasst:
Erhalten, durch die Blutdruckmessvorrichtung (600), der Medikationsinformationen von einer Medikationserinnerungsanwendung; oder
Bestimmen, durch die Blutdruckmessvorrichtung (600), der Medikationsinformationen basierend auf einer Benutzereingabe.

7. Verfahren nach einem der Ansprüche 1 bis 6, wobei das Erhalten, durch die Blutdruckmessvorrichtung (600), der Bewegungsinformationen der getesteten Person ebenso Folgendes umfasst:
Bestimmen, durch die Blutdruckmessvorrichtung (600), der Bewegungsinformationen ebenso basierend auf einer Benutzereingabe.

8. Verfahren nach Anspruch 4, wobei die verwandten Informationen die Schlafinformationen umfassen, die eine Schlafzeit und/oder eine Schlafqualität umfassen.

9. Verfahren nach Anspruch 8, wobei das Erhalten, durch die Blutdruckmessvorrichtung (600), der Schlafinformationen der getesteten Person Folgendes umfasst:
Bestimmen, durch die Blutdruckmessvorrichtung (600), der Schlafinformationen basierend auf Informationen, die durch den Beschleunigungssensor (602), den Kreiselsensor (603) und einen Photoplethysmographie(PPG)-Sensor (604) ausgegeben werden, die an der Blutdruckmessvorrichtung (600) angeordnet sind.

10. Blutdruckmessvorrichtung (600), die einen Prozessor (601) und einen Speicher (610) umfasst, wobei
wobei der Prozessor (601) eine Anweisung ausführt, die in dem Speicher (610) gespeichert ist, um die folgenden Abläufe durchzuführen:
Erhalten von ersten physiologischen Statusinformationen und zweiten physiologischen Statusinformationen einer getesteten Person, wobei die ersten physiologischen Statusinformationen verwandte Informationen sind, die eine Änderung des Blutdrucks der getesteten Person innerhalb eines ersten Zeitraums bewirken, nachdem der Blutdruck kalibriert wird, und die zweiten physiologischen Statusinformationen verwandte Informationen sind, die eine Änderung des Blutdrucks der getesteten Person innerhalb eines zweiten Zeitraums bewirken, bevor der Blutdruck kalibriert wird; und
Bestimmen eines Blutdruckkalibrierungszeitraums basierend auf einem Änderungsgrad der ersten physiologischen Statusinformationen relativ zu den zweiten physiologischen Statusinformationen,
wobei die verwandten Informationen Bewegungsinformationen umfassen, die die folgenden Informationen umfassen: Bewegungsintensität, Bewegungsdauer und/oder eine Bewegungsregelmäßigkeit,
wobei das Erhalten, durch die Blutdruckmessvorrichtung (600), der Bewegungsinformationen der getesteten Person Folgendes umfasst:
Bestimmen, durch die Blutdruckmessvorrichtung (600), der Bewegungsinformationen basierend auf Informationen, die durch einen Beschleunigungssensor (602) und/oder einen Kreiselsensor (603) ausgegeben werden, der/die an der Blutdruckmessvorrichtung (600) angeordnet ist/sind, und
die Blutdruckmessvorrichtung (600) ferner ein Kommunikationsmodul (609) beinhaltet, so dass die Blutdruckmessvorrichtung (600) konfiguriert ist, um mit einer oder mehreren anderen elektronischen Einrichtungen oder einem oder mehreren anderen Servern bidirektional zu kommunizieren, wobei das Kommunikationsmodul (609) konfiguriert ist, um ein Nahfeldkommunikationsprotokoll oder die Nahfeldkommunikation oder ein Kommunikationsprotokoll zu unterstützen, das durch ein Netzwerk verwendet wird.

11. Blutdruckmessvorrichtung (600) nach Anspruch 10, wobei das Bestimmen des Blutdruckkalibrierungszeitraums basierend auf dem Änderungsgrad der ersten physiologischen Statusinformationen relativ zu den zweiten physiologischen Statusinformationen Folgendes umfasst:
Bestimmen, ob der Änderungsgrad der ersten physiologischen Statusinformationen relativ zu den zweiten physiologischen Statusinformationen größer als oder gleich einem voreingestellten Schwellenwert ist; und
falls der Änderungsgrad größer als oder gleich dem voreingestellten Schwellenwert ist, Bestimmen, dass der Blutdruckkalibrierungszeitraum ein erster Zeitraum ist, wobei der erste Zeitraum kleiner als ein voreingestellter Blutdruckkalibrierungszeitraum der Blutdruckmessvorrichtung (600) ist.

12. Blutdruckmessvorrichtung (600) nach Anspruch 10, wobei die Abläufe Folgendes umfassen:
falls der Änderungsgrad kleiner als der voreingestellte Schwellenwert ist, Bestimmen, dass der Blutdruckkalibrierungszeitraum ein zweiter Zeitraum ist, wobei der zweite Zeitraum größer als oder gleich dem voreingestellten Blutdruckkalibrierungszeitraum ist.

13. Blutdruckmessvorrichtung (600) nach einem der Ansprüche 10 bis 12, wobei die verwandten Informationen ebenso folgende Informationen umfassen:
Medikationsinformationen, Schlafinformationen und/oder
Umgebungsinformationen.

14. Blutdruckmessvorrichtung (600) nach Anspruch 13, wobei die verwandten Informationen die Medikationsinformationen umfassen, die eine Arzneimittelart und Arzneimittelkonsuminformationen umfassen, und die Arzneimittelkonsuminformationen folgende Informationen umfassen: eine Arzneimitteldosis, eine Wirksamkeitszeit und/oder eine Medikationsregelmäßigkeit.

## Revendications

1. Procédé de détermination de la période d'étalonnage de la tension artérielle, comprenant :
l'obtention (101), par un dispositif de mesure de la tension artérielle (600), des premières informations d'état physiologique et des secondes informations d'état physiologique d'une personne testée, les premières informations d'état physiologique étant des informations connexes qui provoquent un changement de la tension artérielle de la personne testée pendant une première période de temps après l'étalonnage de la tension artérielle, et les secondes informations d'état physiologique étant des informations connexes qui provoquent un changement de la tension artérielle de la personne testée pendant une seconde période de temps avant l'étalonnage de la tension artérielle ; et
la détermination (102), par le dispositif de mesure de la tension artérielle (600), d'une période d'étalonnage de la tension artérielle en fonction d'un degré de changement des premières informations d'état physiologique par rapport aux secondes informations d'état physiologique,
les informations connexes comprenant des informations d'exercice qui comprennent au moins l'une des informations suivantes : l'intensité de l'exercice, la durée de l'exercice ou une régularité de l'exercice,
l'obtention (101), par le dispositif de mesure de la tension artérielle, des informations d'exercice de la personne testée comprenant :
la détermination, par le dispositif de mesure de la tension artérielle (600), des informations d'exercice sur la base d'informations émises par un capteur d'accélération (602) et/ou un capteur gyroscopique (603) qui sont/est disposé(s) sur le dispositif de mesure de la tension artérielle (600), et
le dispositif de mesure de la tension artérielle (600) comporte en outre un module de communication (609), de telle sorte que le dispositif de mesure de la tension artérielle (600) communique de manière bidirectionnelle avec un ou plusieurs autres appareils ou serveurs électroniques, le module de communication (609) prenant en charge un protocole de communication en champ proche, ou une communication en champ proche, ou un protocole de communication utilisé par un réseau.

2. Procédé selon la revendication 1, dans lequel la détermination (101), par le dispositif de mesure de la tension artérielle (600), de la période d'étalonnage de la tension artérielle en fonction du degré de changement des premières informations d'état physiologique par rapport aux secondes informations d'état physiologique comprend :
la détermination (203), par le dispositif de mesure de la tension artérielle (600), du fait de savoir si le degré de changement des premières informations d'état physiologique par rapport aux secondes informations d'état physiologique est supérieur ou égal à un seuil prédéfini ; et
si le degré de changement est supérieur ou égal au seuil prédéfini, la détermination (403) du fait que la période d'étalonnage de la tension artérielle est une première période, la première période étant inférieure à une période d'étalonnage de la tension artérielle prédéfinie du dispositif de mesure de la tension artérielle.

3. Procédé selon la revendication 2, dans lequel le procédé comprend en outre :
si le degré de changement est inférieur au seuil prédéfini, la détermination (404) du fait que la période d'étalonnage de la tension artérielle est une seconde période, la seconde période étant supérieure ou égale à la période d'étalonnage de la tension artérielle prédéfinie.

4. Procédé selon l'une quelconque des revendications 1 à 3, dans lequel les informations connexes comprennent au moins une des informations suivantes :
des informations sur la médication, des informations sur le sommeil ou des informations sur l'environnement.

5. Procédé selon la revendication 4, dans lequel les informations connexes comprennent les informations sur la médication qui comprennent un type de médicament et des informations d'utilisation de médicaments, et les informations d'utilisation de médicaments comprennent au moins l'une des informations suivantes : une dose de médicament, un temps d'efficacité ou une régularité de médication.

6. Procédé selon la revendication 5, dans lequel l'obtention, par le dispositif de mesure de la tension artérielle (600), des informations sur la médication de la personne testée comprend :
l'obtention, par le dispositif de mesure de la tension artérielle (600), des informations sur la médication à partir d'une application de rappel de médication ; ou
la détermination, par le dispositif de mesure de la tension artérielle (600), des informations sur la médication sur la base d'une entrée utilisateur.

7. Procédé selon l'une quelconque des revendications 1 à 6, dans lequel l'obtention, par le dispositif de mesure de la tension artérielle (600), des informations d'exercice de la personne testée comprend également :
la détermination, par le dispositif de mesure de la tension artérielle (600), des informations d'exercice également sur la base d'une entrée utilisateur.

8. Procédé selon la revendication 4, dans lequel les informations connexes comprennent les informations de sommeil qui comprennent un temps de sommeil et/ou la qualité du sommeil.

9. Procédé selon la revendication 8, dans lequel l'obtention, par le dispositif de mesure de la tension artérielle (600), des informations de sommeil de la personne testée comprend :
la détermination, par le dispositif de mesure de la tension artérielle (600), des informations de sommeil en fonction des informations émises par le capteur d'accélération (602), le capteur gyroscopique (603) et un capteur photopléthysmographique, PPG, (604) qui sont disposés sur le dispositif de mesure de la tension artérielle (600).

10. Dispositif de mesure de la tension artérielle (600), comprenant un processeur (601) et une mémoire (610), dans lequel
dans lequel le processeur (601) exécute une instruction stockée dans la mémoire (610) pour effectuer les opérations suivantes :
l'obtention des premières informations d'état physiologique et des secondes informations d'état physiologique d'une personne testée, les premières informations d'état physiologique étant des informations connexes qui provoquent un changement de la tension artérielle de la personne testée pendant une première période de temps après l'étalonnage de la tension artérielle, et les secondes informations d'état physiologique étant des informations connexes qui provoquent un changement de la tension artérielle de la personne testée pendant une seconde période de temps avant l'étalonnage de la tension artérielle ; et
la détermination d'une période d'étalonnage de la tension artérielle sur la base d'un degré de changement des premières informations d'état physiologique par rapport aux secondes informations d'état physiologique,
les informations connexes comprenant des informations d'exercice, qui comprennent au moins l'une des informations suivantes : l'intensité de l'exercice, la durée de l'exercice ou une régularité de l'exercice,
l'obtention, par le dispositif de mesure de la tension artérielle (600), des informations d'exercice de la personne testée comprenant :
la détermination, par le dispositif de mesure de la tension artérielle (600), des informations d'exercice sur la base d'informations émises par un capteur d'accélération (602) et/ou un capteur gyroscopique (603) qui sont/est disposé(s) sur le dispositif de mesure de la tension artérielle (600), et
le dispositif de mesure de la tension artérielle (600) comporte en outre un module de communication (609), de telle sorte que le dispositif de mesure de la tension artérielle (600) est configuré pour communiquer de manière bidirectionnelle avec un ou plusieurs autres appareils ou serveurs électroniques, le module de communication (609) étant configuré pour prendre en charge un protocole de communications en champ proche, ou une communication en champ proche, ou un protocole de communications utilisé par un réseau.

11. Dispositif de mesure de la tension artérielle (600) selon la revendication 10, dans lequel la détermination de la période d'étalonnage de la tension artérielle en fonction du degré de changement des premières informations d'état physiologique par rapport aux secondes informations d'état physiologique comprend :
la détermination du fait de savoir si le degré de changement des premières informations d'état physiologique par rapport aux secondes informations d'état physiologique est supérieur ou égal à un seuil prédéfini ; et
si le degré de changement est supérieur ou égal au seuil prédéfini, la détermination du fait que la période d'étalonnage de la tension artérielle est une première période, la première période étant inférieure à une période d'étalonnage de la tension artérielle du dispositif de mesure de la tension artérielle (600).

12. Dispositif de mesure de la tension artérielle (600) selon la revendication 10, dans lequel les opérations comprennent :
si le degré de changement est inférieur au seuil prédéfini, la détermination du fait que la période d'étalonnage de la tension artérielle est une seconde période, la seconde période étant supérieure ou égale à la période d'étalonnage de la tension artérielle prédéfinie.

13. Dispositif de mesure de la tension artérielle (600) selon l'une quelconque des revendications 10 à 12, dans lequel les informations connexes comprennent également au moins l'une des informations suivantes :
des informations sur la médication, des informations sur le sommeil ou des informations sur l'environnement.

14. Dispositif de mesure de la tension artérielle (600) selon la revendication 13, dans lequel les informations connexes comprennent les informations sur la médication qui comprennent un type de médicament et des informations d'utilisation de médicaments, et les informations d'utilisation de médicaments comprennent au moins l'une des informations suivantes : une dose de médicament, un temps d'efficacité ou une régularité de médication.
